# EUROPEAN PATENT SPECIFICATION

(11) **EP 2 170 872 B1**
(45) Date of publication and mention of the grant of the patent: **01.09.2010**
(21) Application number: 08795969.8
(22) Date of filing: 23.06.2008
(51) Int. Cl.: C07D 409/12, C07D 333/36

(54) **PROCESS FOR THE PREPARATION OF THE N-(2-CHLORO-4-METHYL-3-THIENYL)-1H- BENZIMIDAZOL-2-AMINE HYDROCHLORIDE AND INTERMEDIATES THEREOF**
VERFAHREN ZUR HERSTELLUNG VON N-(2-CHLOR-4-METHYL-3-THIENYL)-1H-BENZIMIDAZOL- 2-AMIN HYDROCHLORID UND ZWISCHENPRODUKTE DAFÜR
PROCÉDÉ DE PRÉPARATION DE L'HYDROCHLORURE DE LA N-(2-CHLORO-4-MÉTHYL-3- THIÉNYL)-1H-BENZIMIDAZOL-2-AMINE ET SES INTERMÉDIAIRES

(30) Priority: 28.06.2007 US 946791 P
(43) Date of publication of application: 07.04.2010
(73) Proprietor: Sanofi-Aventis U.S. LLC, Bridgewater, NJ 08807 (US)
(72) Inventor: AYERS, Timothy, Allen, Bridgewater, New Jersey 08801 (US); CHOY, Nakyen, Bridgewater, New Jersey 08807 (US); GILL, Harpal, S., Bridgewater, New Jersey 08807 (US); HILLEGASS, Andrea, Bridgewater, New Jersey 08807 (US); SHAY, John, J., Jr., Bridgewater, New Jersey 08807 (US); DERDAU, Volker, D-65929 Frankfurt Am Main (DE)
(74) Representative: Venne-Dunker, Sabine
(86) International application number: PCT/US2008/067851
(87) International publication number: WO 2009/006066

(56) References cited:
- US-B2- 7 049 333

## Description

### Field of the Invention

The present invention relates generally to the preparation of pharmaceutical actives and improvements in the processes and intermediate compounds prepared therein. More specifically, the present invention relates to improvements in the synthesis of a class of sodium/proton exchanger. (NHE) inhibitors whereby improved yields and purity of the final product are achieved. Even more specifically, the present invention relates to improved processes for the synthesis of a class of NHE-3 inhibitors which are useful in the treatment and therapy of sleep apnea.

### Background of the Invention

Sleep apnea or "apnoea" is a respiratory disorder characterized by pauses in breathing during sleep. These episodes, called apneas (literally, "without breath") each last long enough so one or more breaths, which normally occur on a steady, rhythmic basis, are missed. These "missed breaths" can occur repeatedly throughout the period of sleep. There are two distinct forms of sleep apnea: central and obstructive. Breathing is interrupted by the lack of breathing effort in Central Sleep Apnea. However, in Obstructive Sleep Apnea, a physical block of airflow despite ones' breathing effort is the cause of the problem. In Mixed Sleep Apnea, both types of events occur. Notwithstanding the type of apnea involved, the individual affected with sleep apnea is rarely (if ever) aware of having difficultly breathing, even upon awakening. Sleep apnea is recognized as a problem by others witnessing the individual during episodes of sleep, and or although not detected first-hand, is suspected because of its deleterious effects on the body (sequelae). The definitive diagnosis of sleep apnea disorder is made by polysomnography.

In either case, an onset of sleep apnea may result in hypoxia wherein the pause in breathing occurs to such an extent that the percentage of oxygen in the blood circulation drops to a lower than normal level. As this occurs, the concentration of carbon dioxide (CO₂) increases to higher than normal levels in the lungs and bloodstream resulting in hypercapnia. Obviously, this can result in severe consequences such as brain or heart damage and even death if not remedied.

It has been known for some time, that substituted thiophene compounds of Structure I set forth below, possess physio-chemical effects on the sodium/proton exchanger of subtype 3 ("NHE-3") which makes the compounds useful in the treatment of respiratory disorders and disorders of the central nervous system. United States Patent No. 7,049,333 to Lang et. al. discloses clonidine-type compounds which possess enhanced NHE-3 inhibitory properties useful in the treatment of respiratory and cardiovascular disorders.

The NHE-3 inhibitors known in the art may be derived from compounds of the acylguanidine type (EP0825178), the norbornylamino type (DE19960204), the 2-guanidino quinazoline type (WO0179186) or of the benzamidine type (WO0121582; WO172742) The NHE-3 inhibitors produced by the improved process of the present invention are defined by compound of Formula II

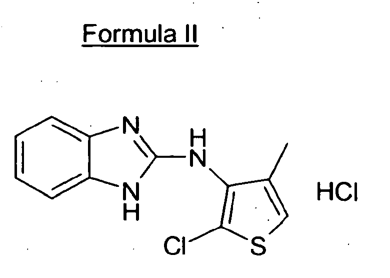

In United States Published Appln. No. 20040242560 to Heinelt et. al.discloses a process in which isothiocyanate of is initially reacted with a base in situ with a primary amine to give a thiourea of formula IV. Subsequently, the thiourea of formula is converted to the corresponding heterocycle using a base and a sulfonyl chloride.

### Summary of the Invention

The present invention is an improved process for the preparation of a sodium/proton exchanger inhibitor of subtype-3 (NHE-3) useful in the treatment of sleep apnea and other related respiratory disorders. The improved synthesis of the NHE-3 universal inhibitor, more specifically a benzimidazol thienylamine, results in an improved yield and purity of the final product with less process steps in the reaction required.

### Detailed Description of the Invention

The present invention is an improved process for the synthesis of a sodium/proton exchanger type-3 (NHE-3), more specifically defined as a benzimidazol thienylamine and derivatives thereof. More specifically, the invention comprised on improved method for the preparation of N-(2-chloro-4-methyl-3-thienyl)-1H-benzimidazol-2-amine hydrochloride as defined by formula II and the derivatives thereof and their precursor intermediate compounds.

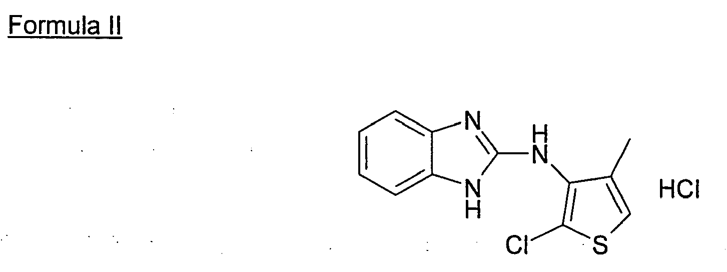

Previously, the production of the NHE-3 inhibitor compound of formula II that is useful in the treatment of sleep apnea and other related respiratory disorders was the product of a number of synthetic steps comprised of cyclization, chlorination and purification. The process described in Lang et al. '333 for example, was comprised of six (6) reaction steps that generally resulted in a low yield with respect to the final product. Previously, the benzimidazol thienylamine compounds and their derivatives were produced by the following prior art reaction scheme I as follows.

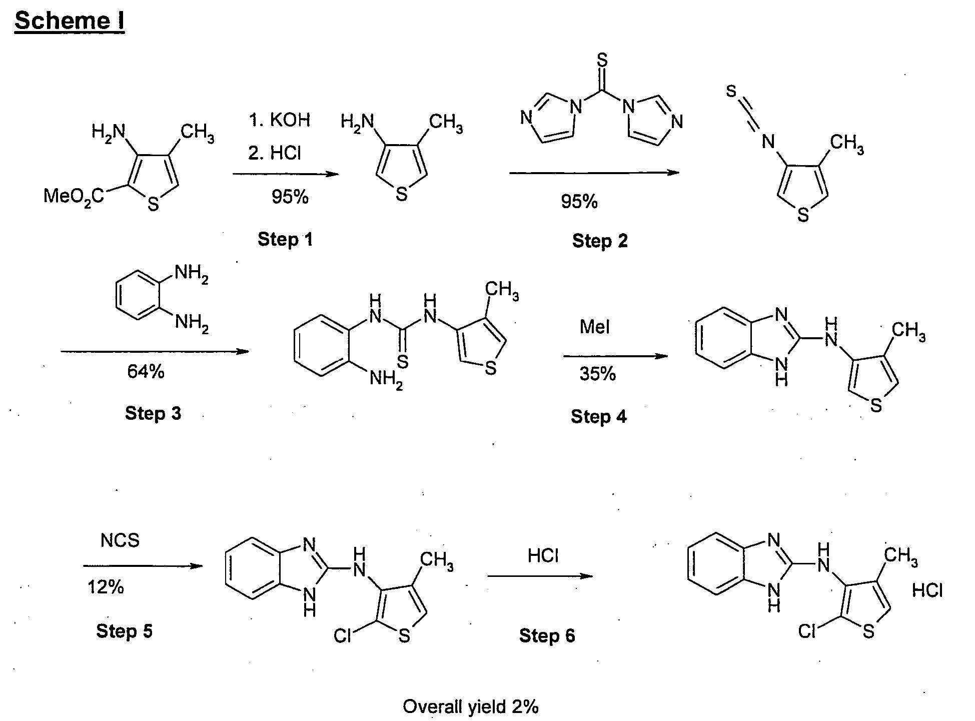

As schematically set forth above, methyl 3-amino-4-methylthiophene-2-carboxylate is de-carboxylated by sequentially treating the compound with:
1) an aqueous first base such as (KOH), (NaOH), or (LiOH) and mixtures thereof, and
2) hydrochloric acid (HCl)
to yield 3-amino-4-methylthiophene. In step 2, the 3-amino-4-methlythiophene is reacted with thiocarbodiimidazole to yield an isothiocyanate. This is then combined with 1,2-phenylenediamine to yield N-(2-aminophenyl)-N'-(4-methyl-3-thienyl)thiourea.

The N-(2-aminophenyl)-N'-(4-methyl-3-thienyl)thiourea is then treated with methyl iodide (CH₃l) to yield N-(4-methyl-3-thienyl)-1H-benzimidazol-2-amine which is then subsequently chlorinated with N-chlorosuccinimide (NCS) that produces N-(2-chloro-4-methyl-3-thienyl)-1H-benzimidazol-2-amine. Finally, the resulting N-(2-chloro-4-methyl-3-thienyl)-1H-benzimidazol-2-amine is converted to the final product N-(2-chloro-4-methyl-3-thienyl)-1H-benzimidazol-2-amine hydrochloride by the addition of hydrogen chloride.

As opposed to prior chemical synthetic methods known in the art, one aspect of the procedure of the present invention comprises a two-fold improvement. On the one hand, in step 4 of the prior art reaction scheme methyl iodide (CH₃l) was used for preparation of the N-(4-methyl-3-thienyl)-1H-benzimidazol-2-amine. As a result, problems existed with respect to the toxicity and volatility of the reaction components. Furthermore, in step 5 of the process of the prior art, wherein the N-(4-methyl-3-thienyl)-1H-benzimidazol-2-amine is converted to N-(2-chloro-4-methyl-3-thienyl)-1H-benzimidazol-2-amine through the addition of NCS (i. e., chlorination occurs late in the process), more impurities in the form of chlorine derivatives are produced resulting in the need for additional chromatographic purification which additionally causes a low overall production yield.

In the present invention as depicted in Scheme II, improvements over the prior art process are realized by replacing the toxic and environmentally hazardous methyl iodide (CH₃l), the cyclization reagent, with a carbodiimide (RNH=C=NHR wherein R=cyclohexyl or isopropyl), or an alkyl or aryl-sulfonyl chloride (RSO₂Cl),such as para-toluene sulfonyl chloride (TsCl) or preferably, benzenesulfonyl chloride (PhSO₂Cl) which better induced the cyclization of N-(2-aminophenyl)-N'-(2-chloro-4-methyl-3-theinyl)thiourea to N-(2-chloro-4-methyl-3-thienyl)-1H-benzimidazol-2-amine.

In the present invention, the ability to carry out the chlorination step earlier in the procedure results in a superior final product purity and yield and furthermore results in the production of novel intermediates. More specifically, in carrying out the improved synthetic route of the present invention, the novel process results in higher yields of final product which does not need to be purified by additional chromatography or distillation steps that are needed in the prior art.
By chlorinating earlier in the reaction process, i. e., in step 2 prior to the formation of the benzimidazole ring, only the thiophene moiety is chlorinated, the benzimidazole ring is not and as a result there is no need for the extensive and time-consuming chromatographic purification steps required later as would otherwise be the case. The process also avoids the production of unstable intermediate compounds during the synthetic pathway that were a direct result of the processes of the prior art. The claimed process of the present invention is also easier to gross up in scale for full manufacturing capability.

In order to carry out the chlorination step at an earlier point in the synthetic process, the methyl 3-amino-4-methylthiophene-2-carboxylate starting material must first be de-carboxylated and the resultant free amine protected by a protecting group such as *N-tert*-butoxycarbonyl [tert-BuOC(O)] by reaction with (tert-BuOCO)₂O. The resulting stable intermediate (4-methyl-thiophen-3-yl) carbamic acid tert-butyl ester is produced in a 70-85% yield and is more amenable to large plant scale-up than the unstable aminothiophene intermediate produced in synthetic route of the prior art.

Schematically; the improved process of the present invention can be set forth as follows.

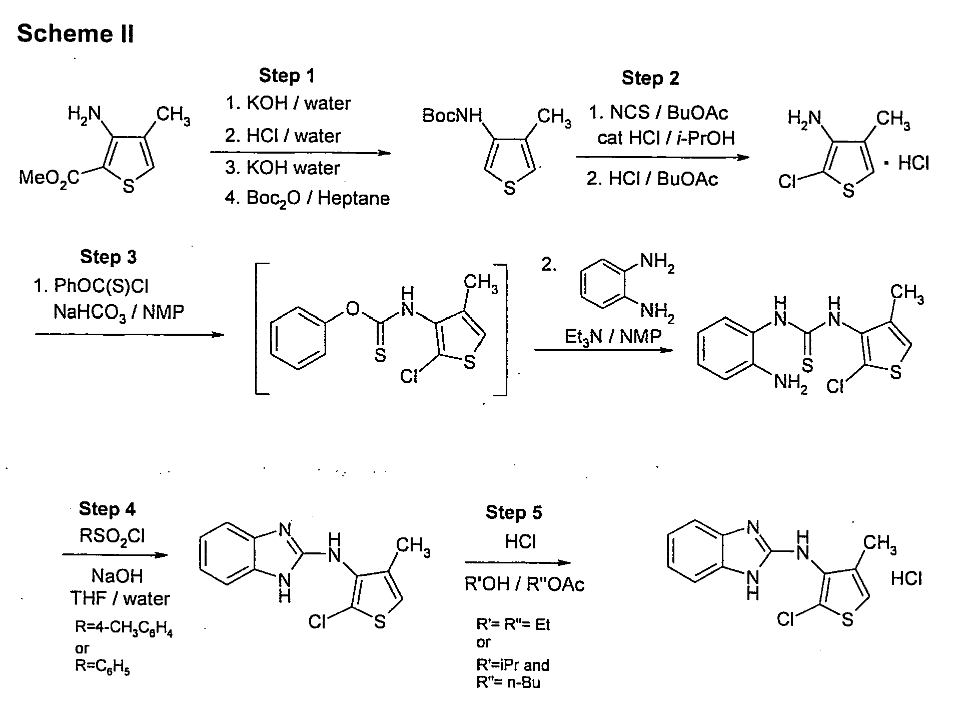

### Step 1

3-Amino-4-methyl-thiophene-2-carboxylic acid methyl ester.
(or) Methyl 3-Amino-4-methyl-thiophene-2-carboxylate.
+ 1) an aqueous first base such as (KOH), (NaOH), or (LiOH) and mixtures thereof,
then 2)HCl → (*DECARBOXYLATION*) → 3-amino-4-methylthiophene
+ (BuOCO)₂O/Heptane → (*PROTECTION*)

### Step 2

(4-Methyl-thiophen-3-yl)-carbamic acid tert-butyl ester
+ N-chlorosuccinamide (NCS) or alternatively, metalation by butyl lithium and reaction with hexachloroethane (Cl₃C₂Cl₃) →
(2-chloro-4-methyl-thiophen-3-yl)-carbamic acid tert-butyl ester
+ HCl or LiCl →

### Step 3

3-amino-2-chloro-4-methylthiophene hydrochloride.
+ aryl chlorothionoformate, preferably phenyl chlorothionoformate →.
(2-chloro-4-methyl-thiophen-3-yl)-thiocarbamic acid O-phenyl ester.
+ 1,2-phenylenenediamine →

### Step 4

N-(2-aminophenyl)-N'-(2-chloro-4-methyl-3-thienyl)thiourea.
+ 1) Carbodiimide (RNH=C=NHR; R=cyclohexyl or isopropyl) or,
   2) CH₃-C₆H₄SO₂Cl/NaOH (TsCl/NaOH) or preferably,
   3) C₆H₅SO₂Cl/NaOH →

### Step 5

N-(2-Chloro-4-methyl-3-thienyl)-1H-benzimidazol-2-amine.
+ 1) HCl in EtOH/EtOAc, or
   2) HCl in Isopropanol/n-BuOAc

   3) HCl in Butanol/n-BuOAc
   →
   N-(2-chloro-4-methyl-3-thienyl)-1H-benzimidazol-2-amine hydrochloride
Optionally, other arylchlorothionoformates or diarylthionocarbonates may be used in reaction step 3 in place of phenyl chlorothionoformate. For example, O-p-tolyl chlorothionoformate or di-2-pyridyl thionocarbonate. The former of the two results in the formation of the novel intermediate 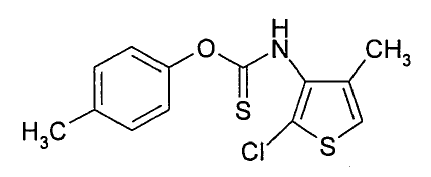 (2-chloro-4-methyl-thiophen-3-yl)-thiocarbamic acid O-p-tolyl ester
While use of di-2-pyridyl thionocarbonate results in the formation of the novel intermediate 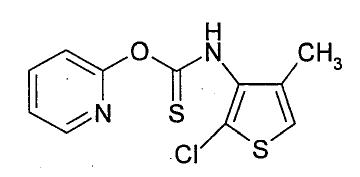 Namely, (2-chloro-4-methyl-thiophen-3-yl)-thiocarbamic acid O-pyridin-2-yl ester

More specifically, in the first step above, methyl 3-amino-4-methylthiophene-2-carboxylate is treated sequentially with aqueous potassium hydroxide followed by the addition of hydrochloric acid to decarboxylate the thiophene. Subsequently, the amino group is protected with di-t-butyldicarbonate to afford the (4-methyl-thiophen-3-yl)-carbamic acid tert-butyl ester.

The (4-methyl-thiophen-3-yl)-carbamic acid tert-butyl ester was then selectively chlorinated with a chlorinating agent such as N-chlorosuccinimide (NCS) and catalytic hydrochloric acid (HCl) or alternatively, by metalation with N-butyl lithium and reaction with hexachloroethane (Cl₃C₂Cl₃) to form 2-chloro-4-methyl-thiophen-3-yl)-carbamic acid tert-butyl ester. This ester is then treated with hydrogen chloride gas to remove the protecting group so as to form the 3-amino-2-chloro-4-methylthiophene hydrochloride. In step 3, the 3-amino-2-chloro-4-methylthiophene hydrochloride is activated for coupling by treatment with phenyl chlorothionoformate and sodium bicarbonate. This intermediate was subsequently treated with 1,2-phenylenediamine and triethylamine to afford N-(2-aminophenyl)-N'-(2-chloro-4-methyl-3-thienyl)thiourea. The thiourea was cyclized utilizing an alkyl or arylsulfonyl chloride such as p-toluenesulfonyl chloride or benzenesulfonyl chloride together with an alkali metal base such as sodium hydroxide (NaOH), sodium carbonate (Na₂CO₃), potassium hydroxide (KOH), lithium hydroxide (LiOH), potassium carbonate (K₂CO₃,) and the like, or alternatively, a carbodiimide to yield the free base of the desired final compound N-(2-chloro-4-methyl-3-thienyl)-1H-benzimidazol-2-amine. The free base was then treated with HCl (g) in ethanol/ethyl acetate or isopropanol/n-butyl acetate or n-butanol/n-butyl acetate to afford the desired compound N-(2-chloro-4-methyl-3-thienyl)-1H-benzimidazol-2-amine hydrochloride.

In this novel route for synthesis, new intermediates are formed and better, purer yields are realized in the production of the final benzimidazole derivative product through:
a. performing the chlorination step earlier in the reaction in step 2;
b. use of an aryl chlorothionoformate selected from the group comprising phenyl chlorothionoformate (PhOC(S)Cl), 4-methylphenyl chlorothionoformate or diaryl thionocarbonates such as di-2-pyridyl thionocarbonate to produce a novel thiocarbamate that more selectively forms the isothiocyanate intermediate in situ thereby providing a higher yield of product;
c. utilization of new cyclization reagents, comprising, a carbodiimide, or preferably, an alkyl or aryl sulfonyl chloride selected from the group comprising benzenesulfonyl chloride, methanesulfonyl chloride or p-toluenesulfonyl chloride
As a result of these key process improvements that avoid the prior art step of chlorination of the benzimidazole ring later on in the process, there is no need for additional chromatographic purification. As a result these processes are more industrially scalable inasmuch as there is no need for the isolation of unstable intermediates which are generated in situ.

One improvement in the present invention is to introduce the chlorine moiety early in the synthesis. In the prior art the thiophene ring is chlorinated late in the synthesis at step 5. This led to chlorination of not only the desired thiophene but other positions on the benzimidazole ring. These chloro - impurities had to be removed by column chromatography. However, by chlorinating prior to the introduction of the benzimidazole ring, no column chromatography was necessary which greatly enhanced the ability to scale up the process.

In order to enhance the early chlorination reaction, the amino group of the 3-amino-4-methyl-thiophene preferably was protected. Thus (4-methyl-thiophen-3-yl)-carbamic acid tert-butyl ester was prepared. This is a novel compound.

Another improvement in the present invention is the use of the reagents aryl chlorothionoformate or diaryl thionocarbonate as activating agents in step 3. The aryl chlorothionoformate may be selected from the group comprising phenyl chlorothionocarbamate (PhOC(S)Cl), 4-methylphenyl chlorothionoformate. The diaryl thionocarbonate may be di-2-pyridyl thionocarbonate. Phenyl chlorothionoformate is preferred.

Whereas the prior art utilized thio-carbodiimidazole to prepare the deschlorothiourea, it was discovered that when 3-amino-2-chloro-4-methylthiophene hydrochloride was treated with thio-carbodiimidazole, it decomposed quickly and lead to more side reactions. The reagent phenyl chlorothionoformate performed much better and when utilized the desired intermediate (2-chloro-4-methyl thiophen-3-yl)-thiocarbamic acid O-phenyl ester was formed and was stable until the next reagents, 1,2-phenylenediame and triethylamine were added. Thus much better control of the formation of the N-(2-aminophenyl)-N'-(2-chloro-4-methyl-3-thienyl)thiourea resulted. This led to higher yields and less impurities. In addition both (2-chloro-4-methyl-thiophen-3-yl)-thiocarbamic acid O-phenyl ester and N-(2-aminophenyl)-N'-(2-chloro-4-methyl-3-thienyl)thiourea are novel compounds.

In the present invention, improvements over the prior art process are also realized by replacing the toxic and environmentally hazardous methyl iodide (CH₃l), the cyclization reagent, with an alkyl or arylsulfonyl chloride (RSO₂Cl),such as p-toluenesulfonyl chloride (TsCl) or preferably benzenesulfonyl chloride (PhSO₂Cl) which better induced the cyclization to N-(2-chloro-4-methyl-3-thienyl)-1H-benzimidazol-2-amine.

In addition the isolation of the N-(2-chloro-4-methyl-3-thienyl)-1H-benzimidazol-2-amine as its HCl salt can be done in a variety of solvents further that can be utilized to remove impurities formed during the synthesis with out the need for column chromatography.

The following examples are provided to more specifically set forth how one skilled in the art may carry out and practice the process and compounds of the present invention. It is to be emphasized however, that they are for illustrative purposes only and describe a number of specific embodiments of the invention.

### Examples

### Preparation of (4-Methyl-thiophen-3-yl)-carbamic acid tert-butyl ester.

A three (3) liter 3-neck round bottom flask equipped with mechanical stirrer, thermocouple, and condenser was charged with methyl 3-amino-4-methylthiophene-2-carboxylate (400g, 2.34 mol) followed by water (800 g) and 45% KOH solution (400 g, 3.28 mol, 1.4 eq.) at room temperature under nitrogen. The suspension was warmed from 30 °C to 80 °C,: After 30 min at 80 °C, the solution was cooled to 20 °C. The contents of the reaction flask were drained into a 2 L flask, providing 1588 g of a yellow solution.
A portion of this yellow solution (1180 g, 1.75. mol) was added slowly to a 50 °C solution of 600 g of water and 37% HCl solution (575 g, 5.84 mol, 3.3 eq.). Once the addition was complete, the mixture was warmed to 55-60° C. Gas evolution was observed. The reaction was cooled to 5 °C. After adding heptane (900 mL), the temperature was adjusted to -10 °C.

A 45% potassium hydroxide solution (707 g, 5.74 mol, 3.2 eq.) was charged to the reactor over 30 min, while keeping the batch temperature less than 10° C. At 5 °C, di-t-butyl dicarbonate (402 g, 1.84 mol, 1.05 eq.) was added. The cloudy orange mixture was warmed to 20 °C. The mixture was stirred at room temperature under nitrogen overnight. The mixture was warmed to 50 °C to provide complete dissolution in the two phases. The aqueous phase was removed. While maintaining the solution at 50 °C, the organic phase was washed with 5% NaHCO₃ (300 mL) and water (300mL). Upon cooling the organic phase to ambient temperature, the product crystallized. Azeodrying was performed under vacuum at 200-250 mbar by simple distillation. The resulting suspension was cooled to 5 °C and the solids were collected by filtration over. The resulting filtercake was washed with cold heptane. After drying in the vacuum oven (45° C) overnight a total of 301 g (81%) of a beige solid was obtained.

### Preparation of 3-N-Boc-amino-2-chloro-4-methylthiophene

A flask with nitrogen purge was charged with (4-methyl-thiophen-3-yl)-carbamic acid tert-butyl ester (1.00 g, 4.69 mmol) and 20 mL THF. The solution was cooled to -78°C and (7.6 mL, 12.2 mmol) n-BuLi was slowly added. After stirring of the reaction mixture for 45 min, (2.88 g, 12.2 mmol) hexachloroethane was added as a solid and the mixture was allowed to warm to room temperature over 10 h. Then 3 mL saturated ammonium chloride solution were added and the phases separated. The aqueous phase was extracted three times with dichloromethane. The combined organic phases were dried over sodium sulfate and concentrated. The crude product was purified by chromatography to provide a colorless solid (590 mg, 51 %).

### Preparation of 3-Amino-2-chloro-4-methylthiophene hydrochloride

A 250 mL-jacketed glass reactor equipped with mechanical stirrer, thermocouple probe and nitrogen purge was charged at room temperature with (4-methyl-thiophen-3-yl)-carbamic acid tert-butyl ester (20 g, 93.8 mmol) followed by n-butyl acetate (150 mL). To the resulting solution was added N-chlorosuccinimide (NCS) (12.8 g, 94.4 mmol, and a hydrochloric acid solution in 2-propanol (IPA) (5.5 N, 435 mg, 2.63 mmol, 2.81 mol%). After 3 h a solution of 2.5 N sodium hydroxide (45.8 g, 103 mmol, 1.1 eq) and sodium hydrogensulfite (560 mg, 5.4 mmoles, 0.06 eq) in water (10 mL) was added. The lower aqueous layer was discarded. The organic phase was washed with water (40 mL). The organic layer was azeodried by distillation under reduced pressure. Hydrogen chloride (15.9 g, 436 mmol, 4.6 eq) was charged to the reactor at 4 ± 4 °C over a period of 30 min using a subsurface tube. A solid precipitated when half of the hydrogen chloride was added. The mixture was warmed to 20 ± 5 °C after 1.5 h, n-butyl acetate (20 mL) was added and the mixture was sparged. The solid was collected by filtration under nitrogen. The filter cake was washed with n-butyl acetate (25 mL) and dried under vacuum at 60 °C and 200 mbar to provide a gray solid, (15.3 g, 88% yield).

### Preparation of N-(2-aminophenyl)-N'-(2-chloro-4-methyl-3-thienyl)thiourea

Phenyl chlorothionoformate (90.6 g, 0.525 mol, 1.05 equiv.) was added to a suspension of NaHCO₃ (46.2 g, 0.55 mol, 1.1 equiv.) in NMP (1.0 L) at 10 °C. 3-Amino-2-chloro-4-methylthiophene hydrochloride (92.0 g, 0.50 mol) was added. The mixture was stirred at 20 °C to produce the intermediate thiocarbamic acid phenyl ester. After 3 h, 1,2-phenylenediamine (70.3 g, 0.65 mol, 1.3 equiv.) was added to the reaction mixture at 5 °C, followed by adding triethylamine (55.7 g, 0.55 mol, 1.1 equiv.). After 3h, the reaction mixture was heated to 30 °C. To the reaction mixture was slowly added water (0.635 L) over 20 min at 30±5 °C, followed by charging additional water (0.365 L). The mixture was stirred overnight at room temperature then cooled to 10 °C. After 1 h at 10 °C, the solid was collected and washed with an NMP-H₂O mixture, followed by water. The solid was dried at 40-45 °C under reduced pressure to give (123.1 g, 82% yield).

### Preparation of N-(2-Chloro-4-methyl-3-thienyl)-1H-benzimidazol-2-amine

A 5-L jacketed glass reactor equipped with mechanical stirrer, thermocouple probe and nitrogen purge was charged at room temperature with N-(2-aminophenyl)-N'-(2-chloro-4-methyl-3-thienyl)thiourea (120.0 g, 0.403 moles) followed by THF (480 mL) and cooled to 3°C. A solution of 50% w/w sodium hydroxide (64.8 g, 0.810 moles) in water (120 mL) was added to this stirred suspension at 3°C. All solids dissolved to give a brown solution. To this solution was added benzenesulfonyl chloride (69.8 g, 0.395 moles) at 3°C. After 30 minutes, the desired product started to precipitate. The resulting solid was collected and washed with a premixed solution of THF/water (120mL THF/ 360 mL water) followed by water (540 mL). The product was dried under vacuum for 16 h to provide an off white solid (99.6 g, 96% yield).

### Preparation of N-(2-Chloro-4-methyl-3-thienyl)-1H-benzimidazol-2-amine hydrochloride

A 1 L round bottom flask was charged with N-(2-Chloro-4-methyl-3-thienyl)-1H-benzimidazol-2-amine (50 g, 0.190 mol) followed by 2-propanol (300 mL) and n-butyl acetate (200 mL) at 22°C. The resultant solution was polish filtered. The flask and filter were rinsed with a 60:40 v/v solution of 2-propanol/n-butyl acetate (50 mL). The filtrates were combined and then charged to a 1 L jacketed reactor. The orange solution was treated with HCl gas (11.1 g, 0.3 mol) at 24 °C. After 30 min, n-butyl acetate (800 mL) was added and a precipitate was observed. Simple vacuum distillation was performed to reduce the volume. The mixture was cooled to 22°C and after 1.5 h, the solid was collected and washed with n-butyl acetate (100 mL) and dried under vacuum for 16 h at 45°C and 200 mbar to provide a tan solid (55.2 g, 97% yield).

## Claims

1. A process for preparing a compound of formula II 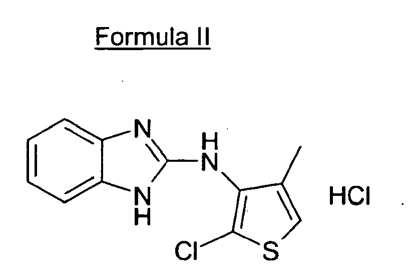 comprising
a) de-carboxylating methyl 3-amino-4-methyl-thiophene-2-carboxylate with an aqueous base followed by the addition of a sufficient amount of an acid to yield 3-amino-4-methylthiophene;
b) protecting said thiophene through the addition of an agent selected from the group consisting of dicarbonates and anhydrides;
c) chlorinating said protected thiophene with a chlorinating agent selected from the group consisting of N-chlorosuccinimide, N-butyl lithium in hexachloroethane (Cl₃C₂Cl ₃) or chlorine gas in the presence of a catalytic amount of hydrochloric acid followed by the treatment with hydrogen chloride gas to remove the tert-butyl ester so as to produce 3-amino-2-chloro-4-methylthiophene hydrochloride;
d) reacting said 3-amino-2-chloro-4-methylthiophene hydrochloride with an aryl chlorothionoformate or di-aryl thionocarbonate and sodium bicarbonate to yield (2-chloro-4-methyl-thiophen-3yl)-thiocarbamic acid O-aryl ester,
e) reacting said (2-chloro-4-methyl-thiophen-3yl)-thiocarbamic acid O-aryl ester with 1 ,2-phenylenediamine in the presence of triethylamine to yield N-(2-aminophenyl)-N'-(2-chloro-4-methyl-3-thienyl)thiourea;
f) cyclizing the 1 N-(2-aminophenyl)-N'-(2-chloro-4-methyl-3-thienyl)thiourea with an alkyl or aryl sulfonyl chloride in the presence of sodium hydroxide (NaOH), sodium carbonate (Na₂CO₃) potassium hydroxide (KOH), lithium hydroxide (LiOH), potassium carbonate (K₂CO₃) or mixtures thereof, or a carbodiimide to yield the free base of N-(2-chloro-4-methyl-3-thienyl)-1H-benzimidazol-2-amine, and
g) acidifying said free base of N-(2-chloro-4-methyl-3-thienyl)-1H-benzimidazol-2-amine with hydrogen chloride in a solvent to yield the compound of formula I.

2. The process of claim 1 wherein said aqueous base of step a) is selected from the group consisting of sodium hydroxide (NaOH), potassium hydroxide (KOH), lithium hydroxide (LiOH), and mixtures thereof.

3. The process of claim 2 wherein said protecting agent of step b) is selected from the group consisting of di-t-butyldicarbonate or trifluoroacetic anhydride.

4. The process of claim 3 wherein said aryl chlorothionoformate of step d) is selected from the group consisting of phenyl chlorothionoformate and O-p-tolyl chlorothionoformate or wherein said di-aryl thionocarbonate is di-2-pyridyl thionocarbonate.

5. The process of claim 4 wherein said alkyl or aryl sulfonyl chloride of step f) is selected from the group consisting of benzenesulfonyl chloride, methanesulfonyl chloride and p-toluenesulfonyl chloride.

6. The process of claim 5 wherein said solvent of step g) is selected from the group consisting of ethanol/ethyl acetate, isopropanol, isopropyl acetate , butanol, butyl acetate, hexanol, hexyl acetate and mixtures thereof.

7. The process of claim 6 wherein said carbodiimide of step f) is selected from the group consisting of compounds defined by the structure RNH=C=NHR wherein R is selected from the group consisting of a cyclohexyl or isopropyl group.

8. The compound of formula III:

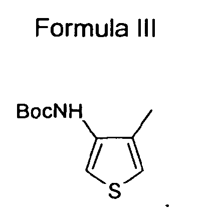

9. The compound of formula IV:

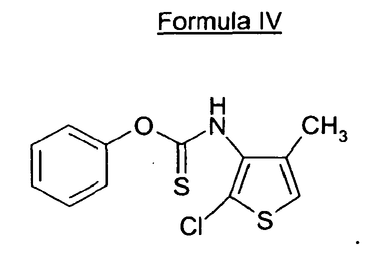

10. The compound of formula V:

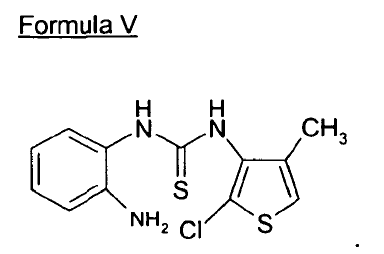

11. The compound of formula VI:

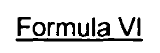

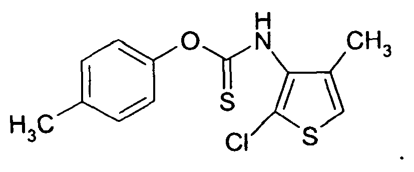

12. The compound of formula VII:

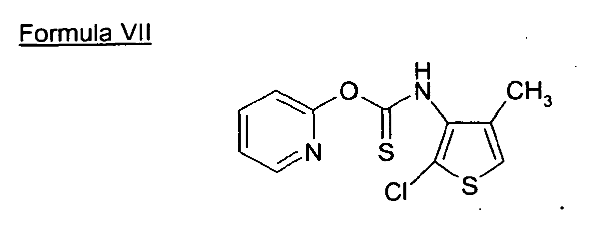

## Patentansprüche

1. Verfahren zur Herstellung einer Verbindung der Formel II 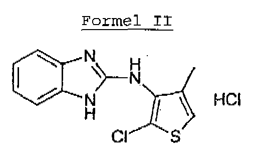 bei dem man
a) 3-Amino-4-methylthiophen-3-carbonsäuremethylester mit einer wäßrigen Base decarboxyliert und dann eine ausreichende Menge einer Säure zugibt, wobei man 3-Amino-4-methylthiophen erhält;
b) das Thiophen durch Zugabe eines Agens aus der Gruppe bestehend aus Dicarbonaten und Anhydriden schützt;
c) das geschützte Thiophen mit einem Chlorierungsmittel aus der Gruppe bestehend aus N-Chlorsuccinimid, N-Butyllithium in Hexachlorethan (Cl₃C₂Cl₃) oder Chlorgas in Gegenwart einer katalytisch wirksamen Menge Salzsäure chloriert und dann zur Entfernung des t-Butylesters mit Chlorwasserstoffgas behandelt, wobei man 3-Amino-2-chlor-4-methylthiophen-hydrochlorid erhält;
d) das 3-Amino-2-chlor-4-methylthiophen-hydrochlorid mit einem Arylchlorthionoformiat oder Diarylthionocarbonat und Natriumhydrogencarbonat zu (2-Chlor-4-methylthiophen-3-yl)thiocarbamidsäure-O-arylester umsetzt;
e) den (2-Chlor-4-methylthiophen-3-yl)thiocarbamidsäure-O-arylester in Gegenwart von Triethylamin mit 1,2-Phenylendiamin zu N-(2-Aminophenyl)-N'-(2-chlor-4-methyl-3-thienyl)thioharnstoff umsetzt;
f) den N-(2-Aminophenyl)-N'-(2-chlor-4-methyl-3-thienyl)thioharnstoff in Gegenwart von Natriumhydroxid (NaOH), Natriumcarbonat (Na₂CO₃), Kaliumhydroxid (KOH), Lithiumhydroxid (LiOH), Kaliumcarbonat (K₂CO₃) oder Mischungen davon oder Carbodiimid mit einem Alkyl- oder Arylsulfonylchlorid zu der freien Base von N-(2-Chlor-4-methyl-3-thienyl)-1H-benzimidazol-2-amin umsetzt und
g) die freie Base von N-(2-Chlor-4-methyl-3-thienyl)-1H-benzimidazol-2-amin mit Chlorwasserstoff in einem Lösungsmittel ansäuert, wobei man die Verbindung der Formel I erhält.

2. Verfahren nach Anspruch 1, bei dem man die wäßrige Base von Schritt a) aus der Gruppe bestehend aus Natriumhydroxid (NaOH), Kaliumhydroxid (KOH), Lithiumhydroxid (LiOH) und Mischungen davon auswählt.

3. Verfahren nach Anspruch 2, bei dem man das Schützungsmittel von Schritt b) aus der Gruppe bestehend aus Di-t-butyldicarbonat oder Trifluoressigsäureanhydrid auswählt.

4. Verfahren nach Anspruch 3, bei dem man das Arylchlorthionoformiat von Schritt d) aus der Gruppe bestehend aus Phenylchlorthionoformiat und O-p-Tolylchlorthionoformiat auswählt oder es sich bei dem Diarylthionocarbonat um Di-2-pyridylthionocarbonat handelt.

5. Verfahren nach Anspruch 4, bei dem man das Alkyl- oder Arylsulfonylchlorid von Schritt f) aus der Gruppe bestehend aus Benzolsulfonylchlorid, Methansulfonylchlorid und p-Toluolsulfonylchlorid auswählt.

6. Verfahren nach Anspruch 5, bei dem man das Lösungsmittel von Schritt g) aus der Gruppe bestehend aus Ethanol/Essigsäureethylester, Isopropanol, Essigsäureisopropylester, Butanol, Essigsäurebutylester, Hexanol, Essigsäurehexylester und Mischungen davon auswählt.

7. Verfahren nach Anspruch 6, bei dem man das Carbodiimid von Schritt f) aus der Gruppe bestehend aus Verbindungen der Struktur RNH=C=NHR, worin R aus der Gruppe bestehend aus einer Cyclohexyl- oder Isopropylgruppe ausgewählt ist, auswählt.

8. Verbindung der Formel III:

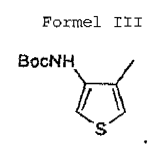

9. Verbindung der Formel IV:

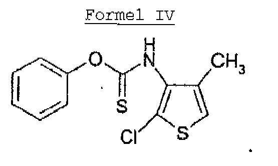

10. Verbindung der Formel V:

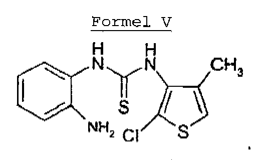

11. Verbindung der Formel VI:

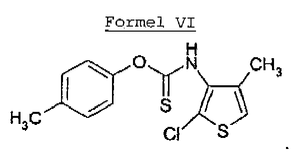

12. Verbindung der Formel VII:

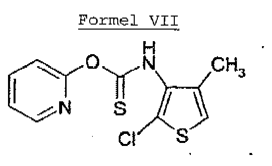

## Revendications

1. Procédé pour préparer un composé de formule II 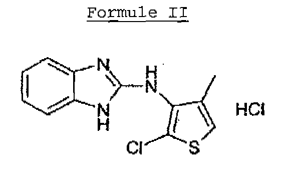 comprenant
a) la décarboxylation de 3-amino-4-méthyl-thiophène-2-carboxylate de méthyle avec une base aqueuse suivie par l'ajout d'une quantité suffisante d'un acide pour obtenir le 3-amino-4-méthylthiophène ;
b) la protection dudit thiophène par l'ajout d'un agent choisi dans le groupe constitué de dicarbanates et d'anhydrides ;
c) la chloration dudit thiophène protégé avec un agent de chloration choisi dans le groupe constitué du N-chlorosuccinimide, du N-butyl-lithium dans de l'hexachloroéthane (Cl₃C₂Cl₃) ou du gaz de chlore en présence d'une quantité catalytique d'acide chlorhydrique suivie par le traitement avec du gaz de chlorure d'hydrogène pour éliminer l'ester tert-butylique de manière à produire le chlorhydrate de 3-amino-2-chloro-4-méthylthiophène ;
d) la réaction dudit chlorhydrate de 3-amino-2-chloro-4-méthylthiophène avec un chlorothionoformate ou thionocarbonate de diaryle et du bicarbonate de sodium pour obtenir l'ester O-arylique d'acide (2-chloro-4-méthyl-thiophén-3-yl)-thiocarbamique ;
e) la réaction dudit ester O-arylique d'acide (2-chloro-4-méthyl-thiophén-3-yl)-thiocarbamique avec de la 1,2-phénylènediamine en présence de triéthylamine pour obtenir la N-(2-aminophényl)-N'-(2-chloro-4-méthyl-3-thiényl)thiourée ;
f) la cyclisation de la 1-N-(2-aminophényl)-N'-(2-chloro-4-méthyl-3-thiényl)thiourée avec un chlorure d'alkyl- ou arylsulfonyle en présence d'hydroxyde de sodium (NaOH), de carbonate de sodium (Na₂CO₃), d'hydroxyde de potassium (KOH), d'hydroxyde de lithium (LiOH), de carbonate de potassium (K₂CO₃) ou des mélanges de ceux-ci, ou un carbodiimide pour obtenir la base libre de N-(2-chloro-4-méthyl-3-thiényl)-1H-benzimidazol-2-amine, et
g) l'acidification de ladite base libre de N-(2-chloro-4-méthyl-3-thiényl)-1H-benzimidazol-2-amine avec du chlorure d'hydrogène dans un solvant pour obtenir le composé de formule I.

2. Procédé de la revendication 1 **caractérisé en ce que** ladite base aqueuse de l'étape a) est choisie dans le groupe constitué de l'hydroxyde de sodium (NaOH), de l'hydroxyde de potassium (KOH), de l'hydroxyde de lithium (LiOH), et de mélanges de ceux-ci.

3. Procédé de la revendication 2 **caractérisé en ce que** ledit agent protecteur de l'étape b) est choisi dans le groupe constitué du carbonate de di-t-butyle ou de l'anhydride trifluoroacétique.

4. Procédé de la revendication 3 **caractérisé en ce que** ledit chlorothionoformate d'aryle de l'étape d) est choisi dans le groupe constitué du chlorothionoformate de phényle et du chlorothionoformate de O-p-to1yle ou **caractérisé en ce que** ledit thionocarbonate de diaryle est le thionocarbonate de di-2-pyridyle.

5. Procédé de la revendication 4 **caractérisé en ce que** ledit chlorure d'alkyl- ou arylsulfonyle de l'étape f) est choisi dans le groupe constitué du chlorure de benzènesulfonyle, du chlorure de méthanesulfonyle et du chlorure de p-toluènesulfonyle.

6. Procédé de la revendication 5 **caractérisé en ce que** ledit solvant de l'étape g) est choisi dans le groupe constitué d'un mélange d'éthariol/acétate d'éthyle, l'isopropanol, l'acétate d'isopropyle, le butanol, l'acétate de butyle, l'hexanol, l'acétate d'hexyle et des mélanges de ceux-ci.

7. Procédé de la revendication 6 **caractérisé en ce que** ledit carbodiimide de l'étape f) est choisi dans le groupe constitué de composés définis par la structure RNH=C=NHR où R est choisi dans le groupe constitué du groupe cyclohexyle ou isopropyle.

8. Composé de formule III :

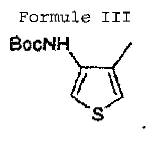

9. Composé de formule IV :

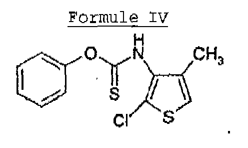

10. Composé de formule V :

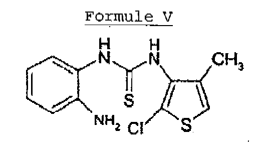

11. Composé de formule VI :

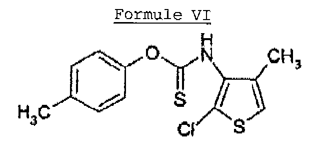

12. Composé de formule VII :
